# EUROPEAN PATENT APPLICATION

(11) **EP 1 227 157 A1**
(43) Date of publication of application: **31.07.2002**
(21) Application number: 01870011.2
(22) Date of filing: 19.01.2001
(51) Int. Cl.: C12N 15/66, C12N 15/861

(54) **Swap/counter selection: a rapid cloning method**

(71) Applicant: Galapagos Genomics B.V., 2301 CA Leiden (NL)
(72) Inventor: Van Es, Helmuth Hendrikus Gerardus, 2133 DJ Hoofddorp (NL); Piest, Ivo, 3645 VS Vinkeveen (NL); Van der Stolpe, Onno, 2311 GB Leiden (NL); Michiels, Godefridus Augustinus Maria, 2352 EP Leiderdorp (NL)
(74) Representative: Hooiveld, Arjen Jan Winfried

(57) **Abstract**

The present invention relates to a method for cloning a sample nucleic acid from a donor vector conferring resistance to one or more antibiotics of a first type to an acceptor vector conferring resistance to one or more antibiotics of a second without purification of said sample nucleic acid from donor vector. After restriction digestion, with appropriate enzymes, of the donor and acceptor vectors, the digests are simply mixed, ligated and transformed into host cells, which are plated and selected for resistance conferred by the acceptor vector. Preferably the method is followed by a further step comprising plating said selected host cells on plates containing selective growth medium containing the antibiotic for which the donor vector confers resistance and discarding colonies which still grow under these counter selection conditions. After restriction digestion, with appropriate enzymes, of the donor and acceptor vectors, the digests are simply mixed, ligated and transformed into host cells, which are plated and selected for resistance conferred by the acceptor vector.

## Description

### Field of the invention

The present invention relates to the field of recombinant DNA technology and describes the procedure of cloning individual nucleic acids from pre-existing collections of donor vectors into desired acceptor vectors, without the need to isolate inserts from the donor vector, thereby allowing the procedure to become amenable to automation and miniaturization. In particular, the invention relates to the use of different selection markers on the donor and the acceptor vector.

### Background

The field of functional genomics relates to high-throughput analysis of gene function by screening large collections of cDNAs in phenotypic assays in order to ascribe potential functions to genes (functional screening). Usually, cDNA libraries consisting of 'random' collections of partial and full-length cDNA clones are used in functional screenings. However, recent initiatives have resulted in the availability of large collections of individual sequence-validated full-length cDNA clones, which provide powerful new reagents for functional screenings.

Unfortunately, the re-cloning of large collections of full-length cDNAs in acceptor vectors is currently impossible in an automated setting, since cDNA clones have to be digested with restriction endonucleases and the inserts have to be purified from the donor vector prior to ligation into the desired acceptor vector. The most laborious part of this procedure is the gel-isolation of the insert cDNAs to separate them from the donor vector, and the subsequent purification of the cDNAs from agarose (Sambrook et al. Molecular Cloning: A laboratory Manual. Cold Spring Harbor Laboratory Press 1989). Although several methods exist for the purification of DNA from agarose or polyacrylamide (PAA), such as phenol extraction, electroelution and purification over glass (beads), none of these methods are compatible with a high-throughput, automated system. Moreover, the loading of the samples on a gel, and the cutting of the desired fragments out of the gel, remains a tedious task that is not easily amenable to automation.

Recently, two methods were described that circumvent the gel-isolation procedure for recloning (cDNA) inserts, Gateway™ from Life Technologies (US5888732) and Echo™ Cloning System from Invitrogen (US5851808). Both methods make use of unique sequences that surround the cDNA inserts, which sequences are recognized by specific enzymes, namely Integrase for the Gateway™ system and Crerecombinase for the Echo™ Cloning System. These enzymes are used to swap the inserts from a donor vector to a compatible acceptor vector. However, as these systems were introduced recently, existing large collections of cDNAs are not compatible with these beforementioned cloning systems.

The aim of the present invention is to provide a rapid re-cloning method for sample nucleic acids to circumvent the problems concerning non-compatibility of the current DNA collections with specific cloning systems.

Further, it is an aim of the present invention to provide preferable cloning vectors particularly designed to be used in a rapid re-cloning method.

### Summary of invention

The present invention provides an alternative method for transferring nucleic acid inserts without purification and makes use of a selection marker method.

The present invention is based on a very simple procedure, and easy to automate in a high-throughput setting, which takes advantage of the fact that multiple resistance markers exist which can be used to select for a specific vector. In this scenario, acceptor vectors are constructed which contain distinct resistance markers such as, but not limited to, ampicillin, tetracycline, chloramphenicol, kanamycin, or zeocin that differ from the resistance marker of the donor vector. After swapping of the nucleic acid insert from the donor vector to the acceptor vector, the acceptor vector is specifically selected using the unique resistance marker of the acceptor vector.

In this setup, the donor vector is digested with restriction endonucleases that free the sample nucleic acid insert from the donor vector backbone. This mixture is directly added to the acceptor vector, without isolation or purification of the insert sample nucleic acid. The acceptor vector is linearized with compatible restriction endonucleases and contains a different resistance marker. Following ligation, the mixture is transformed into a suitable host and plated on selection plates containing the antibiotic for which the acceptor vector provides resistance. In this way, only clones containing the acceptor vector will survive.

The whole procedure can be done in a multiwell format, i.e. on 6, 24, 96 or 384 and even 1536 well plates, and is easy to automate. Most of the procedure can be done in succession on the same plate, simply by adding the next reagents and, therefore, does not require any sophisticated robotics.

### Detailed description of the Invention

The present invention relates more particularly to a method for cloning a sample nucleic acid from a donor vector conferring resistance to one or more antibiotics of a first type to an acceptor vector conferring resistance to one or more antibiotics of a second type without purification of said sample nucleic acid from said donor vector, comprising the following steps:
(a) digesting said donor vector comprising said sample nucleic acid to be cloned with at least one restriction endonuclease which releases said sample nucleic acid from said donor vector,
(b) digesting said acceptor vector with at least one compatible restriction endonuclease for cloning said sample nucleic acid,
(c) adding together the product of step (a) and (b) in a ligation reaction mixture,
(d) transformation of said mixture of step (c) into a suitable host,
(e) plating said host cells of step (d) on plates containing selective growth medium containing one or more antibiotics of the second type, and,
(f) possibly isolating vector nucleic acid containing said sample nucleic acid from the host cells resulting after step (e).

In a preferred embodiment step (e) of said method of the invention is followed by a further step comprising plating said selected host cells of step (e) on plates containing selective growth medium containing the antibiotic for which the donor vector confers resistance and discarding colonies which still grow under these counter selection conditions.

In a preferred embodiment said sample nucleic acid is part of a set or library of sample nucleic acids that have beenindividually cloned into the donor vector.

In a preferred embodiment said method of the invention is part of an automated or high-throughput procedure.

In a preferred embodiment said donor vector used in a method of the invention as defined above contains an ampicillin-resistance gene.

In a preferred embodiment said acceptor vector used in a method of the invention as defined above contains a zeocin-resistance gene.

In a preferred embodiment said acceptor vector used in a method of the invention as defined above is an expression vector.

In a more preferred embodiment said acceptor vector is a viral expression vector.

In an even more preferred embodiment said acceptor vector is a retroviral vector, and most preferred said acceptor vector is an adenoviral vector.

In a preferred embodiment said adenoviral vector is an adenoviral adapter vector which contains the left ITR and part of the E2B region, and in which the E1 region has been exchanged for a mammalian promoter, a polylinker sequence, and a polyadenylation signal.

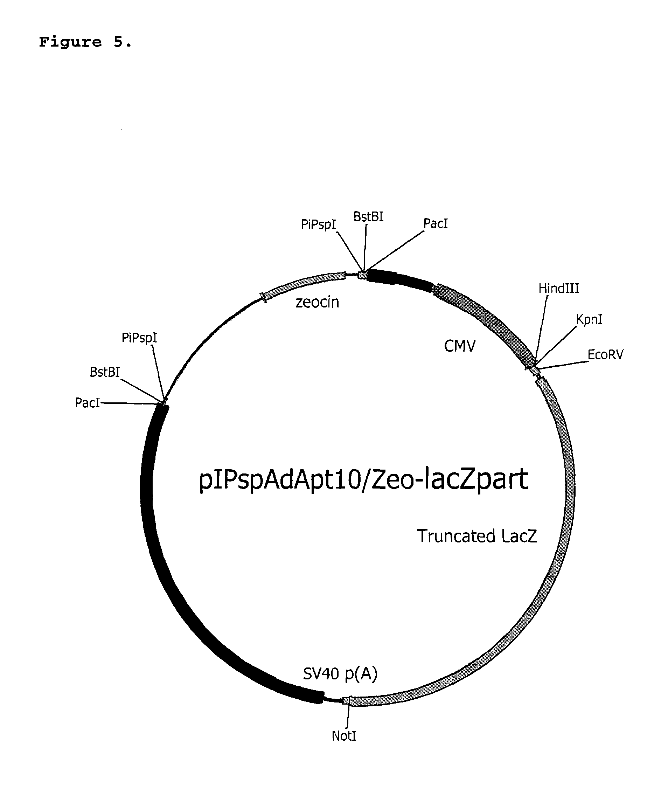

In a more preferred embodiment said adenoviral vector is pIPspAdAptlO/Zeo-laczpart as shown in Figure 5 which was produced as described in detail in the Examples section.

In a further embodiment the present invention also relates to an adenoviral vector pIPspAdApt10/Zeo-lacZpart as shown in Figure 5.

Most cloning vectors used today contain the ampicillin resistance gene for selection in *Escherichia coli.* The recloning of inserts from one vector to another is routinely performed by digestion of donor and acceptor vectors with compatible restriction endonucleases, isolation of the donor insert and acceptor vector by agarose or polyacrylamide (PAA) gel electrophoresis, followed by purification of the desired DNA fragments from the agarose or PAA gel. The purified DNA fragments are subsequently ligated together, the ligation mixture is transformed into *Escherichia coli,* and the bacteria are plated on selective, ampicillin-containing medium to select for bacteria that have received a copy of the vector. Subsequently, independent colonies are inoculated and analyzed for the presence of the insert into the acceptor vector. In this regard, the terms "purified" and "purification" relate to the physical separation of the sample nucleic acid from the donor vector, thus digestion or liberation of the sample nucleic acid from the donor vector is not intended.

However, with the use of different selection markers in vectors to be used in a method according to the invention as set out above, it is possible to swap inserts from donor vector to acceptor vector, without the need to purify the donor insert fragment (sample nucleic acid) from the donor vector fragment. Selection markers that can be used in vectors to be used in a method according to the present invention refer to genes conferring antibiotic resistance and include, but are not limited to, ampicillin-, chloramphenicol-, kanamycin-, zeocin- and tetracyclin-resistance genes, conferring resistance to the respective antibiotics. A vector comprising a specific type of selection marker can grow under conditions containing the respective antibiotic to which said selection marker confers resistance.

In the setup according to the present invention, a donor vector, conferring resistance to one of the above mentioned antibiotics, i.e. an antibiotic of the first type, is digested with restriction endonucleases that release the insert from the vector. An acceptor vector, conferring resistance to one of the other above mentioned antibiotics, i.e. an antibiotic of the second type, is digested with compatible restriction endonucleases and added together with the digested donor vector in a ligation reaction mixture. Following transformation of the ligation mixture into a suitable host, such as, for example, bacteria, e.g. *E. coli,* the host cells are plated on plates containing selective growth medium, i.e. containing one or more antibiotics of the second type. For example, the bacteria are plated on LB-agar plates that contain the antibiotic for which the acceptor vector confers resistance. In this way, only host cells, e.g. bacteria that have been transformed with the acceptor vector can form colonies. The selection of bacteria or other host cells containing an antibiotic resistance marker of the second type can also take place in liquid medium instead of plates. If needed, vector nucleic acid containing said sample nucleic acid from the host that results after the aforementioned procedure can be isolated.

Reference is made to an antibiotic of a "first type" and an antibiotic of a "second type" to refer to two different types of antibiotic resistance markers present in the donor and acceptor vectors to be used in a method according to the present invention so that selection and possibly also counterselection can take place.

The donor vector to be used in a method according to the present invention can be any vector into which a sample nucleic acid has been inserted, including but not limited to vectors containing a bacterial origin of replication and resistance marker, bacterial phage derived vectors such as lambda-, M13-, P1-vectors, and yeast vectors such as yeast artificial chromosomes (YAC).

Sample nucleic acids to be re-cloned in a method according to the present invention are cDNA, genomic DNA, previously cloned DNA, genes, ESTs, synthetic oligonucleotides, randomized sequences, antisense nucleic acids, genetic suppressor elements, ribozymes, mutant zinc fingers, antibody sequences or any combination thereof. In a preferred embodiment, the sample nucleic acid is a cDNA encoding a full-length protein, but it might also encode a partial, not full length, protein. In an even more preferred embodiment, the sample nucleic acid is part of a set or library of sample nucleic acids that are individually cloned into the donor vector, whereby the set or library of sample nucleic acids consists of between 2 and 2x10⁷ individual sample nucleic acids, and usually between 100 and 1x10⁶ individual sample nucleic acids, whereby each individual nucleic acid is contained in a separate compartment, such as individual well in a multiwell plate.In a most preferred embodiment, the complete sequence of the set or library of sample nucleic acids is known and can be used to determine the strategy for liberating the inserts comprising the set or library of sample nucleic acids from the donor vector.

The acceptor vector to be used in a method according to the present invention can be any vector in which the set or library of sample nucleic acids has to be inserted. In a preferred embodiment the acceptor vector is an expression vector, such as, for example, a vector for high level expression in *E. coli*, a mammalian expression vector, an insect expression vector, a plant expression vector, or a yeast two-hybrid vector. In a more preferred embodiment, the acceptor vector is a viral expression vector, such as, for example, a herpes virus vector, a baculovirus vector, a lentivirus vector, an adeno-associated virus vector, a retrovirus vector, an alphavirus vector, or any combination thereof. Retroviral vectors that can be used include, but are not limited to, Moloney Murine Leukemia Virus (Mo-MuLV)-derived viral vectors such as the pBabe (Morgenstern JP and Land H (1990). Nucleic Acids Res., 18, 3587-3596), deltaMoPyF101 (Havenga MJE et al. (1997) Gene Therapy, 4, 1393-1400) or pMFG vectors. Dranoff G et al. (1993) Proc Natl Acad Sci USA, Apr 15; 90, (8):3539-43). In an even more preferred embodiment, the acceptor vector is an adenoviral vector. In the most preferred embodiment, the acceptor vector is an adenoviral adapter vector (e.g. pClip, pAdApt, pIPspAdApt) which contains the left ITR and part of the E2B region, and in which the E1 region has been exchanged for a mammalian promoter, a polylinker sequence, and a polyadenylation signal. Preferably, at least two acceptor vectors are generated that contain different resistance markers to be compatible with different donor vectors.

Large batches of digested acceptor vector, using all combinations of restriction endonucleases, can be prepared in advance. Batches which show little or no religation in the absence of donor vectors should be used preferentially for the swap/counter selection procedure. However, it is contemplated that the acceptor vector can be digested simultaneously or after digestion of the donor vector. Hence, the donor vector and the acceptor vector can be digested in the same reaction vessel.

The term "compatible restriction endonuclease" as used in step (b) of a method according to the invention as defined above refers to restriction endonucleases (type II) that generate compatible cohesive termini compared to the termini generated in step (a) of the method of the present invention. The restriction endonuclease recognizes specific DNA sequences of dyad symmetry (restriction sites), and cleaves each strand of the substrate DNA on the 5' end or the 3' end of the axis of dyad symmetry, resulting in staggered DNA breaks. These breaks yield fragments of DNA that either carry protruding cohesive 5' or protruding cohesive 3' termini, respectively. In addition, certain restriction endonucleases cleave both strands of DNA at the axis of dyad symmetry producing blunt ended termini. Hence, compatible cohesive termini are termini that either form hydrogen bonded basepairs (protruding cohesive 5' and protruding cohesive 3' termini) if they are incubated under conditions that favor formation of basepairs, or are blunt ended termini, which can be joined together with ligase, such as, for example T4 DNA ligase. It will be understood that cleaved restriction sites can be partially filled in with the deoxyribonucleotide of choice by DNA polymerase to generate compatible termini. It will be understood by the person skilled in the art that the term "compatible restriction endonuclease" also relates to isoschizomers. Digestion is the cleavage of the double stranded substrate DNA by a restriction endonuclease.

The sample nucleic acid can be liberated from the donor vector using restriction endonucleases that cut on both sites of the sample nucleic acid. These restriction endonucleases may have additional sites within the vector, but preferably not within the sample nucleic acid. In general, restriction endonucleases that have been used to generate the donor vector can be used to liberate the sample nucleic acid. However, individual members of a set or library of sample nucleic acids may have internal sites, e.g. when they have been constructed with the help of so-called adapters. An example of this is the use of EcoRI adapters during the generation of cDNA libraries (Stratagene). In that case, the donor vector must contain sites for other restriction endonucleases between the vector and the sample nucleic acid. The sequence of the set or library of sample nucleic acids can then be used to group the set or library of sample nucleic acids according to the restriction endonucleases that can be used to liberate the individual donor sample nucleic acids from the vector.

In the setup of the present invention it is preferred to use those restriction endonucleases that will produce either two different protruding cohesive termini, or one blunt ended terminus and one protruding cohesive terminus, to allow directional cloning as will be clear to those skilled in the art.

Digestion of the donor vector containing the set or library of sample nucleic acids can be performed in microcentrifuge tubes, in a 96 well plate, in a 384 well plate or even in a 1536 well plate. In a preferred scenario, between 0.01 and 0.5 microgram of donor vector is restricted with the appropriate restriction endonucleases for 2 hours at the optimal temperature in a final volume of between 2.5 microliter and 5 microliter. Digestion in multiwell plates can be done in an automated setting by using 96 or 384 channel pipettors such as a Multimek (Beckman) or a Quadra (Tomcat), and multiwell dispensers like, for example, a multidrop.

The acceptor vector, which has been digested with appropriate restriction endonucleases to create compatible termini, is added to the digested donor vector in 10x concentrated ligation buffer (e.g. New England Biolabs) such that the final concentration of ligation buffer is 1x. This is done also to inactivate the restriction endonucleases that were used to digest the donor vector and possibly the acceptor vector, since most restriction endonucleases are inactive in 1x ligation buffer. Again, this can be done in an automated setting by using 96 or 384 channel pipettors such as a Multimek (Beckman) or a Quadra (Tomcat), and multiwell dispensers like, for example, a multidrop. Restriction endonucleases that are still active in 1x ligation buffer can be inactivated prior to the addition of the acceptor vector, by incubation at 65 °C for one hour, or by incubation at -20 °C for 16 hours, depending on whether or not they can be heat inactivated, respectively. It will be understood by thoseskilled in the art that by using isoschizomers to generate the compatible termini, inactivation of the isoschizomers is not necessary per se. The preferred volume for digestion is between 1 and 100 microliter, and more preferably between 5 and 20 microliter.

Following addition of the digested acceptor vector, ligation is carried out. Ligation is the formation of phosphodiester bonds between adjacent 3'-hydroxyl and 5'-phosphate termini in DNA, in this way DNA molecules with compatible termini can be joined. Preferentially, ligation is carried out for 2 hours at room temperature using for instance T4 DNA ligase or any other suitable DNA ligase as will be known to those experienced in the art. Ligation can be done in an automated setting by using 96 or 384 channel pipettors such as a Multimek (Beckman) or a Quadra (Tomcat), and multiwell dispensers like, for example, a multidrop. Following ligation, an aliquot of the ligation reaction mixture is used to transform suitable host cells. In particular, an aliquot of the ligation mixture between 0.1 and 10 microliter, or more preferably between 1 and 2 microliter, is taken, added to a new plate and kept on ice. Between 10 and 50 microliters of suitable host cells, such as, for example competent *E. coli* bacteria (e.g. DH5-alpha, subcloning efficiency; Life Technologies) are added, followed by incubation on ice for 30 minutes, and heat shock at 37 °C for 1 minute. Again, this can be done in an automated setting by using 96 or 384 channel pipettors such as a Multimek (Beckman) or a Quadra (Tomcat), and multiwell dispensers like, for example, a multidrop. It will be clear for those skilled in the art, that any suitable host cells as well as any suitable host cell transformation procedure known in the art, amenable to automation, can be used in a method according to the present invention.

After transformation the bacteria are plated on selection media (LB-agar) containing the antibiotic for which the acceptor vector confers resistance, i.e. of the second type, followed by an overnight incubation at 37 °C.

As will be clear for those experienced in the art, single colonies are subsequently used to inoculate minipreps in the presence of the antibiotic for which the acceptor vector confers resistance. These small-scale overnight cultures are subsequently used to isolate vector DNA for analysis, such as sequence- or restriction analysis. The present inventors have found that a considerable percentage (up to 50 %) of the acceptor vector will contain the donor vector inserted into the acceptor vector, which are false positives. To reduce the amount of false positives in the DNA minipreps, and to reduce the amount of labour, all colonies are also streaked out on plates containing the antibiotic for which the donor vector confers resistance, i.e. antibiotics of the first type. A colony that grows on the plate containing the antibiotic for which the donor vector confers resistance will not be used further, e.g. for miniprep DNA isolation. By this procedure, defined as "counter selection", the percentage of correctly swapped donor inserts is considerably enhanced.

Most of the method of the present invention is easy to miniaturize and automate, allowing high-throughput conversion of large sets of donor inserts into adenoviral acceptor vectors. Large sets of donor inserts may comprise between 2 and 2x10⁷ different sample nucleic acids, and usually will contain between 100 and 1x10⁶ different sample nucleic acids. Miniaturization and automation allows conversion of these large sets of sample nucleic acids at a rate of between 10 and 1x10⁴ different sample nucleic acids per day, and usually between 96 and 6144 different sample nucleic acids per day, and is therefore defined as high-throughput conversion.

The availabily of two types of acceptor vectors that differ in their polylinker orientation such as pIPspAdApt8/Zeo and pIPspAdApt10/Zeo, which are described in the examples, will allow the rapid cloning of a set or library of sample nucleic acids in the sense and anti-sense orientation. Anti-sense expression has proven to be a valuable tool for knock down strategies.

A set or library of sample nucleic acids that were cloned into expression vectors according to the method described in the present invention, can be used to generate transducing vectors. For example, active recombinant viruses can be generated by introducing viral vectors - containing the sample nucleic acid- in their respective packaging cell lines such as retroviral vectors in BOSC23 cells (Pear WS et al. (1993), Proc. Natl. Acad. Sci.,90, 8392-8396), or Phoenix cells (Grignani F (1998), Cancer Res.,jan 1;58(1):14-9) and adenovirus vectors (WO99/64582) in for example the PerC6 packaging cell line (US5994128). Sets or libraries of the abovementioned transducable vectors can be generated in a high-throughput fashion and used in high-throughput functional screening methods, basically as described in WO99/64582. Chimeric adenovirus vectors (WO00/03029) or adeno-associated virus vectors (WO/99/32647) can also be used for such high-throughput screening procedures.

The following examples and figure merely serve to illustrate the present invention and are in no way to be contrued as limiting the present invention.

### Examples

### Example 1: Construction of adenoviral acceptor vectors

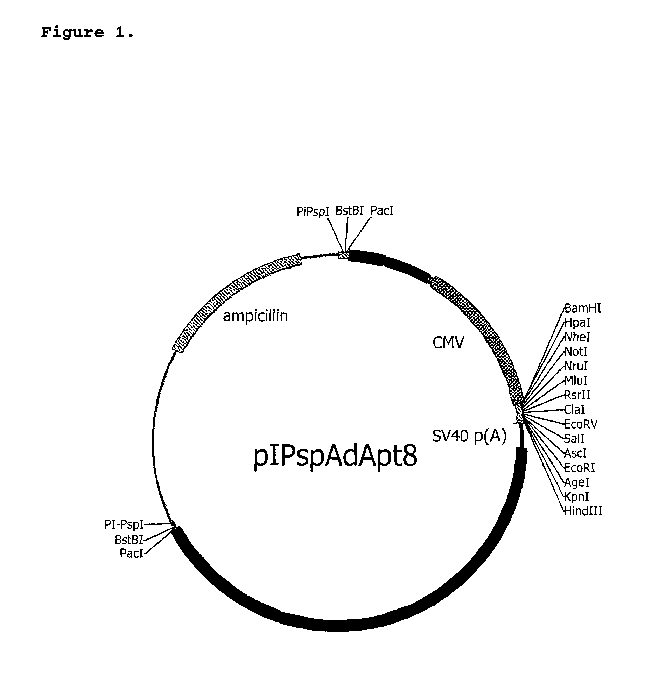

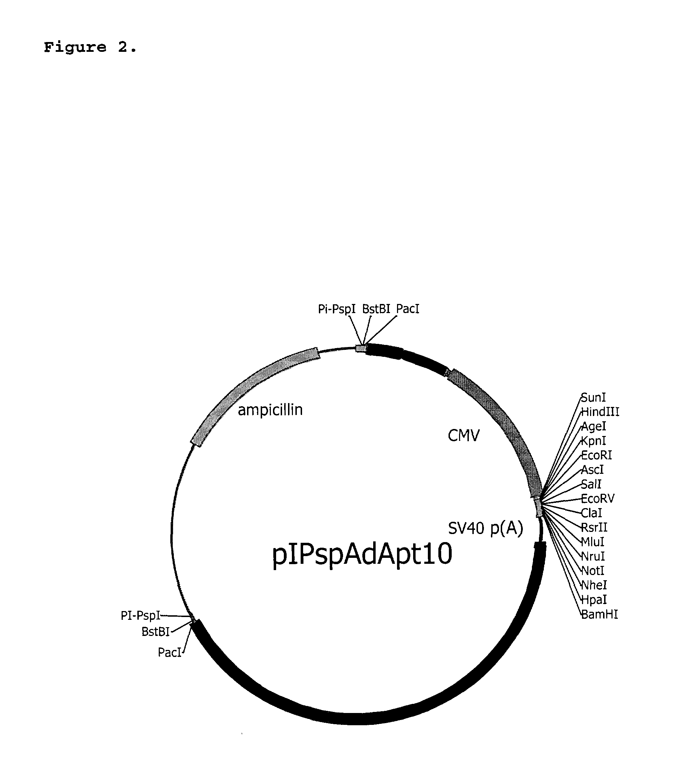

The basis of the present invention is the use of adenoviral acceptor vectors that confer resistance to different antibiotics after transformation to a suitable host cell, such as, for example, *E. coli.* We have previously described the generation of the adenoviral adapter vectors pIPspAdApt (1-7), which all confer ampicillin resistance after transformation to *E. coli* (WO 99/64582). Several additions were made to these adapter plasmids. pIPspAdApt8 (Figure 1) was constructed by digestion of pIPspAdApt6 with HindIII and BamHI. The liberated polylinker was isolated over gel, purified by a Qiaquick gelextraction kit (Qiagen), and cloned into HindIII-BamHI-digested pIPspAdApt4. In this way, the orientation of the polylinker is inverted compared to pIPspAdApt6. To generate pIPspAdApt10, pIPspAdApt6 was digested with HindIII and EcoRI. The oligo's SUNIFOR: 5'-AGCTCGTACGAAGCTTGGTACCGGTG-3' (SEQ ID NO 1) and SUNIREV: 5'-AATTCACCGGTACCAAGCTTCGTACG-3' (SEQ ID NO 2) were annealed by heating for 1 minute at 96 °C and cooling overnight to room temperature, followed by ligation into HindIII-EcoRI-digested pIPspAdApt6. In this way, the original HindIII site of pIPspAdApt6 is destroyed, and a SunI site, followed by a new HindIII site and the remaining of the pIPspAdApt6 polylinker are inserted, leading to pIPspAdApt10 (Figure 2).

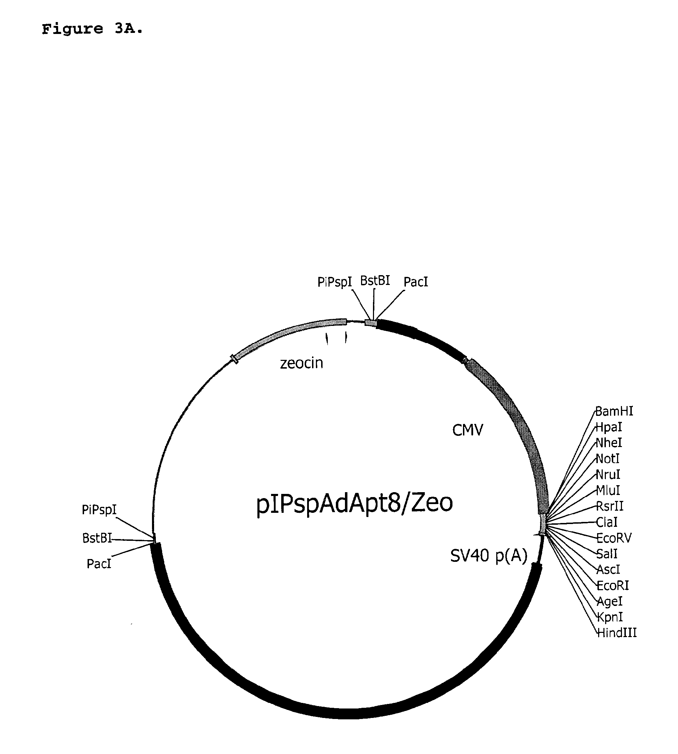

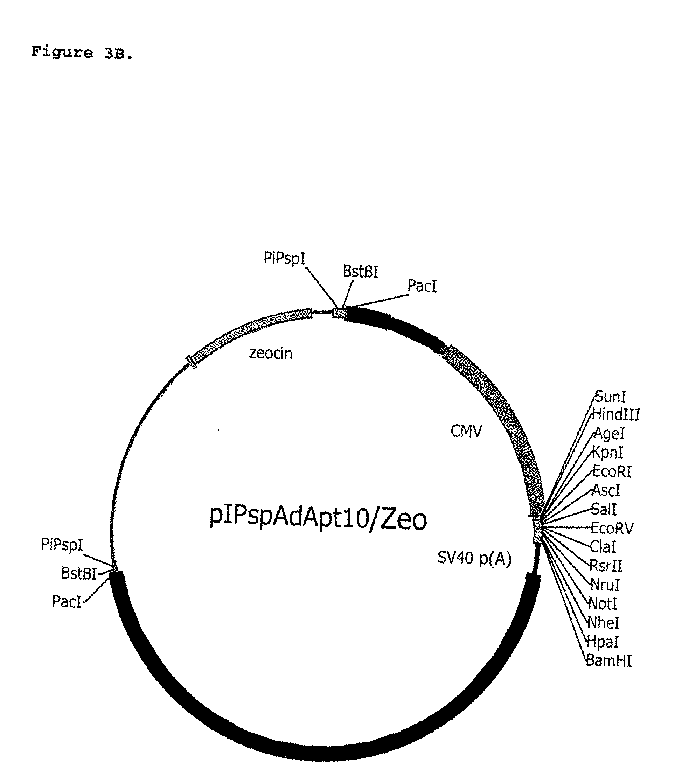

The adenoviral sequences surrounding the ampicillin resistance gene and the bacterial origin of replication of pIPspAdApt6 were amplified by PCR using Elongase (Life Technologies) and primers SspI-forward (CCGAAAAGTGCCACCTGACG, SEQ ID NO 3) and DraI-reverse (TAAAAGGATCTAGGTGAAGATCC, SEQ ID NO 4). The PCR fragment was subsequently treated with T4 polymerase (Life Technologies) to blunt the ends of the fragment. A restriction fragment containing the *Sh ble* gene was isolated from pZeoSV2+ (InVitrogen) by digestion with KspI and MaeI, followed by treatment with T4 polymerase to blunt the ends of the fragment. Subsequently, the PCR fragment was ligated to the fragment containing the *Sh ble* gene and, after transformation in *E. coli,* zeocin-resistant colonies were selected on LB-Zeocin plates. Correct clones were identified by restriction analyses of miniprep DNA. As most of the resulting vector was derived from an *in vitro* amplified fragment, and therefore might contain errors, we wanted to ensure that especially the adenoviral part of the vector contained the correct sequences. Therefore, one of the correct DNAs, isolated by the miniprep procedure (miniprep DNA) was digested with Pad, and the resulting 2.2 kb fragment, containing the bacterial origin of replication and the *Sh ble* gene, was ligated to the 4.1 kb PacI-PacI fragments of pIPspAdApt8 and pIPspAdApt10. The latter fragments contain the adenoviral part of the adapter vectors but differ in their polylinker sequences. Following transformation into *E. coli* and selection of zeocin-resistant colonies, correct clones were identified by restriction analyses of miniprep DNA. The final vectors were designated pIPspAdApt8/Zeo and pIPspAdApt10/Zeo (Figures 3A+B). The sequences surrounding the *Sh ble* gene were verified by sequence analyses.

### Example 2: Generation of universal adenoviral acceptor vector

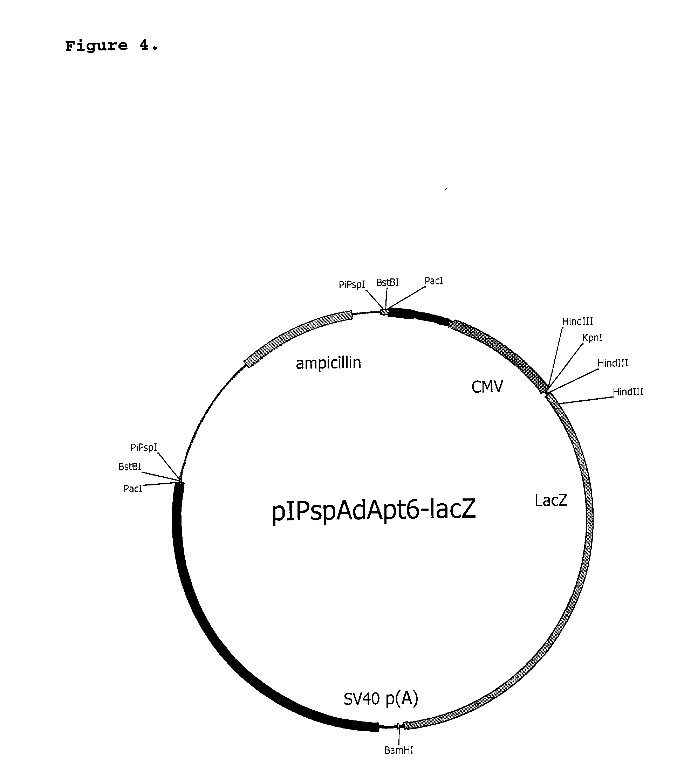

To lower the background of religated acceptor vector, due to incomplete digestion of the acceptor vector, a large part of the *lacZ* gene was inserted into the NruI site of pIPspAdApt10/Zeo (Figure 3B) to create a universal adenoviral acceptor vector (Figure 5). The *lacZ* gene is present in pIPspAdApt6-lacZ (Figure 4), which was constructed by digestion of pIPspAdApt6 with KpnI and BamH1, followed by insertion of the correspondingly digested and purified nls-*lacZ* gene from pCLIP-lacZ (WO 00/52186). The *lacZ* gene was isolated by HindIII-EcoRI digestion of pIPspAdApt6-lacZ, blunt-ended by addition of Klenow enzyme (Life Technologies) and deoxyribonucleotides (Amersham-Pharmacia), and ligated into NruI-digested and dephosphorylated pIPspAdApt10/Zeo. Following transformation into *E*. *coli,* a correct clone named pIPspAdApt10/ZeolacZpart (Figure 5) was selected by restriction analysis.

Using the universal adenoviral acceptor vector, pIPspAdApt10/Zeo-lacZpart, the correctly digested acceptor vector, resulting from digestion with one restriction endonuclease that digests in front of the *lacZ* gene and one restriction endonuclease that digests behind the *lacZ* gene, can be isolated following separation by agarose gel electrophoresis, as it differs in molecular weight from the *lacZ* insert and the singular digested acceptor vector.

### Example 3: Feasibility of swap procedure

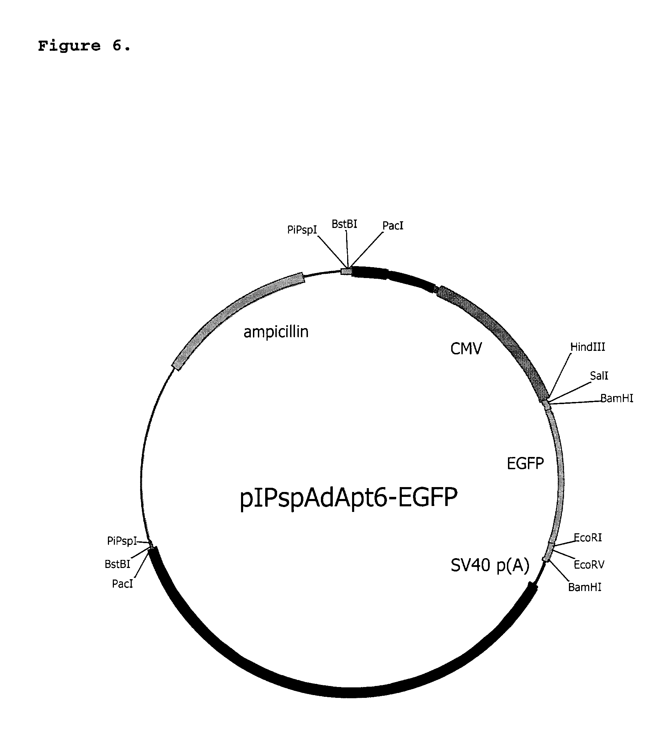

To show the feasibility of the swap procedure, 10 microgram of the pZeo.SV2+ vector (Invitrogen), conferring Zeocin resistance, was digested with the restriction endonucleases EcoRI and HindIII. The vector was gel purified over a 0.8% agarose gel, and DNA was isolated using the QIAquick PCR purification method (pZEO/gel). As an alternative, 10 microgram of pZeo.SV2+ vector (Invitrogen) was treated for 15 minutes at 37 °C and 15 minutes at 65 °C with Shrimp Alkaline Phosphatase (SAP; Roche) after digestion with EcoRI and HindIII (pZEO/SAP). As donor, we used pIPspAdApt6-EGFP. pIPspAdApt6-EGFP, conferring ampicillin resistance, was constructed by releasing the EGFP insert by HindIII-EcoRI digestion from the plasmid pEGFP (Clontech; catalogus number 6077-1), followed by insertion into HindIII/EcoRI-digested pIPspAdApt6 to generate pIPspAdApt6-EGFP (Figure 6). 10 microgram of pIPspAdApt6-EGFP was digested with EcoRI and HindIII to release the EGFP insert from the vector. Half of the digest was loaded on an agarose gel and the EGFP insert was purified using QIAquick PCR purification method (Qiagen). 2.5 microgram of the remaining 5 microgram was treated with Shrimp Alkaline Phosphatase (SAP; Roche) for 15 minutes at 37 °C and 15 minutes at 65 °C. The remaining 2.5 microgram were not treated further. Aliquots of all fragments/digests were run on an agarose gel and stained with Ethidium Bromide to compare the amounts of DNA.

Ligations were set up for 4.5 hours at room temperature in a total volume of 10 microliter using 0.5 Units of T4 DNA ligase in 1x ligation buffer (Life Technologies) to compare the SAP-treated versus the gel-purified pZeo.SV2+ vector. As donors, the HindIII/EcoRI-digested pIPspAdApt6-EGFP (EGFP) was compared to the SAP-treated, HindIII/EcoRI-digested pIPspAdApt6-EGFP (EGFP/SAP), and to the gel-purified HindIII/EcoRI EGFP insert (EGFP/gel). Two molar ratios of pZeo.SV2+ acceptor vector and donor EGFP insert were used in the ligations: 1:1 and 3:1.

1 microliter of all ligation reactions was transformed to MAX efficiency Stbl2 competent cells (Life Technologies), according to the protocol of the manufacturer. Following transformation, cells were plated on a LB-agar-Zeocin plate. 10 colonies were picked from all plates containing the non-purified EGFP as donor and 5 colonies from the plates containing the gel-purified EGFP donor fragment. Miniprep DNA was digested by EcoRI and HindIII to analyze the presence of the 3.5 kb pZeo.SV2+ vector and the 0.7 kb EGFP fragment.

The following results were obtained for the different acceptor (ZEO/SAP and ZEO/gel) and donor (EGFP, EGFP/gel and EGFP/SAP) combinations:
Ratio 1:1 (pZEO/SAP : EGFP)
   8 out of 10 colonies show the correct restriction pattern
Ratio 3:1 (pZEO/SAP : EGFP)
   4 out of 10 colonies show the correct restriction pattern
Ratio 1:1 (pZEO/SAP : EGFP/gel)
   5 out of 5 colonies show the correct restriction pattern
Ratio 3:1 (pZEO/SAP : EGFP/gel)
   5 out of 5 colonies show the correct restriction pattern
Ratio 1:1 (pZEO/gel : EGFP/SAP)
   9 out of 10 colonies show the correct restriction pattern
Ratio 3:1 (pZEO/gel : EGFP/SAP)
   8 out of 10 colonies show the correct restriction pattern
Ratio 1:1 (pZEO/gel : EGFP/gel)
   5 out of 5 colonies show the correct restriction pattern
Ratio 3:1 (pZEO/gel : EGFP/gel)
   5 out of 5 colonies show the correct restriction pattern
Conclusion: The swap cloning procedure works.In this set up, a molar excess of SAP-treated acceptor vector is not preferred.

To further optimize the swapping efficiency, the ratios of gel-purified HindIII/EcoRI-digested Zeo.SV2+ acceptor (pZEO/gel) and SAP-treated HindIII/EcoRI-digested PIPspAdApt6-EGFP donor (EGFP/SAP) were further modified. The fragments used were identical to those mentioned above.
Ratio 1:2 (pZEO/gel : EGFP/SAP)
   10 out of 10 minipreps selected on Zeocin-containing plates and grown in Zeocin-containing LB are correct
Ratio 2:1 (pZEO/gel : EGFP/SAP)
   8 out of 10 minipreps selected on Zeocin-containing plates and grown in Zeocin-containing LB are correct
Ratio 5:1 (pZEO/gel : EGFP/SAP)
   9 out of 10 minipreps selected on Zeocin-containing plates and grown in Zeocin-containing LB are correct
Conclusion: there is no clear relation between the acceptor:donor ratio and the efficiency of swapping. The overall efficiency of gel-purified acceptor is good.

In a next experiment, the gel-purified, HindIII/EcoRI-digested pZeo.SV2+ acceptor (pZEO/gel) was ligated in different ratios to HindIII/EcoRI-digested pIPspAdApt6-EGFP, which was not treated with SAP (EGFP):
Ratio 1:1 (pZEO/gel: EGFP)
   17 out of 20 minipreps selected on Zeocin-containing plates and grown in Zeocin-containing LB are correct
Ratio 1:3 (pZEO/gel:EGFP)
   16 out of 20 minipreps selected on Zeocin-containing plates and grown in Zeocin-containing LB are correct
Ratio 3:1 (pZEO/gel:EGFP)
   14 out of 20 minipreps selected on Zeocin-containing plates and grown in Zeocin-containing LB are correct
Conclusion: SAP treatment of donor DNA is not required

**Table 1.**

| *Summary of the different donor (EGFP) and acceptor (ZEO) ligation combinations (correct clones*/*tested clones)* | | | | |
|---|---|---|---|---|
| Ratio acceptor/donor: | acceptor: | donor: | donor: | donor: |
| | | EGFP/gel | EGFP/SAP | EGFP |
| 1:1 | pZEO/gel | 5/5 | 9/10 | 17/20 |
| 1:2 | pZEO/gel | | 10/10 | |
| 1:3 | pZEO/gel | | | 16/20 |
| 2:1 | pZEO/gel | | 8/10 | |
| 3:1 | pZEO/gel | | 8/10 | 16/20 |
| 5:1 | pZEO/gel | 5/5 | 9/10 | |
| 1:1 | PZEO/SAP | 5/5 | | 8/10 |
| 3:1 | PZEO/SAP | 5/5 | | 4/10 |

General conclusion: Overall efficiency of gel-purified acceptor is good. SAP treatment of donor DNA is not required. Again, there is no clear relation between acceptor:donor ratio and efficiency of swapping, although a ratio of 1:1 seems optimal.

### Example 4: Insert swapping with adenoviral acceptor vector

To show the feasibility of the swapping procedure with an adenoviral acceptor vector, pIPspAdapt10/Zeo-lacZpart was digested with SalI and NotI in React 3 (Life Technologies) and the linearized vector band was isolated over a 0.8% agarose gel and purified using a Gene Clean Spin kit (Bio101). Donor vectors were derived from a human leucocyte cDNA library in pSport, which confers ampicillin resistance (obtained from Life Technologies). Miniprep DNA from 4 different cDNA clones, with insert sizes of about 0.6 kilobases (kb), 1.6 kb, 2 kb, and 3 kb, as judged by estimation of the insert sizes on a 0.8% agarose gel, was also digested with SalI and NotI for 2 hours at 37°C, followed by an overnight storage at -20°C. Prior to ligation, the digests were put for 40 minutes at 65 °C to inactivate the restriction endonucleases. An aliquot of digested donor and purified acceptor DNA was run on a 0.8% agarose gel to compare the staining intensities of the donor insert bands and the acceptor vector band by ethidium bromide staining.

Equal molar amounts, as judged from the staining intensities, of donor insert, in the presence of donor vector, and acceptor vector were ligated in 5 microliter containing 1x ligation buffer and 1.0 Unit of T4 DNA ligase (Life Technologies) for 3.5 hours at room temperature. 1 microliter of this ligation mixture was transformed to subcloning efficiency DH5α cells (Life Technologies) according to the protocol of the manufacturer, and cells were plated on an LB plate containing 50 µgram/ml zeocin (Invitrogen). All transformations yielded over 150 colonies on the LB-zeocin plates.

10 colonies of each ligation were inoculated in LB-zeocin medium and grown overnight at 37°C. Following miniprep DNA isolation, miniprep DNA was digested with SalI and NotI, and run on a 0.8% agarose gel.

Of the 10 colonies which were derived from the swapping of the 0.6 kb insert, 8 colonies yielded correct pIPspAdapt10/Zeo vectors from which the 0.6 kb insert could be released with digestion by SalI and NotI.

Of the 10 colonies which were derived from the swapping of the 1.6 kb insert, 7 colonies yielded correct pIPspAdapt10/Zeo vectors from which the 1.6 kb insert could be released with digestion by SalI and NotI.

Of the 10 colonies which were derived from the swapping of the 2 kb insert, 8 colonies yielded correct pIPspAdapt10/Zeo vectors from which the 2 kb insert could be released with digestion by SalI and NotI.

Of the 10 colonies which were derived from the swapping of the 3 kb insert, 5 colonies yielded correct pIPspAdapt10/Zeo vectors from which the 3 kb insert could be released with digestion by SalI and NotI.

Thus, the overall efficiency was 28/40 = 70% of correctly swapped sample nucleic acids.

### Example 5: Swap/counter selection

We noted that most of the false positive colonies that grew on LB-zeocin showed a similar restriction pattern after miniprep analysis, and suspected that this could be due to ligation of the donor vector in the acceptor vector. To test this, several of the false positive colonies were streaked out on an ampicillin-containing LB-agar and a zeocin-containing LB-agar plate. Most of them indeed were able to grow on both ampicillin plates and zeocin plates. This made it possible to use the ability to grow on ampicillin as a way to counter select for false positives in the swap procedure.

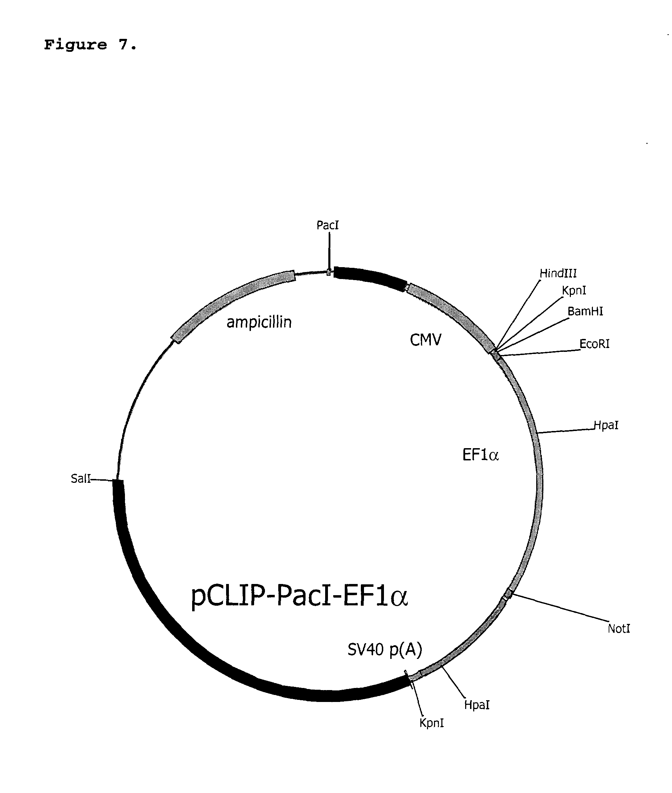

To further proof the use of counter selection, a cDNA encoding human Elongation Factor 1α (EF1α derived from the human leucocyte cDNA library in pSport), was isolated by SalI and NotI digestion, followed by treatment with T4 polymerase (Life Technologies) in the presence of dNTP's to generate blunt ends. The insert was cloned into the adenoviral adapter vector pCLIP-PacI (WO 99/55132) that was digested with EcoRV and treated with Shrimp Alkaline Phosphatase (Life Technologies) to diminish religation of the vector. A correct pCLIP-PacI-EF1α (Figure 7) as judged by restriction analysis and confirmed by sequence analysis, was digested by MluI and NotI for 2 hours and stored at -20 °C.

Two independently generated batches of MluI-NotI digested universal pIPspAdapt10/Zeo-lacZpart acceptor vector (Example 2), termed batch 1 and batch 2, were isolated over an agarose gel and purified using a Gene Clean Spin DNA isolation kit from BIO101.

Ligations were set up in a final volume of 10 microliter containing 1x ligation buffer and 10 Units of T4 ligase (New England Biolabs). The amounts of digested pCLIP-PacI-EF1α donor and purified pIPspAdapt10/ZeolacZpart acceptor were such that the molar ratio of donor insert to acceptor vector was 1:1, as judged by comparing the staining intensities of the donor insert bands and the acceptor vector band.

Ligations were carried out for 24 hours at 12°C, followed by transformation into subcloning efficiency DH5α competent cells of a 1 microliter aliquot of the ligation mixture. After transformation, cells were plated on zeocin-containing LB-agar plates. 30 colonies of each transformation were subsequently streaked out on both ampicillin- and zeocin-containing LB-agar plates.

Of the 30 colonies derived from acceptor batch 1, 9 colonies grew also on ampicillin-containing plates. Of the 30 colonies derived from acceptor batch 2, 12 colonies grew also on ampicillin-containing plates.

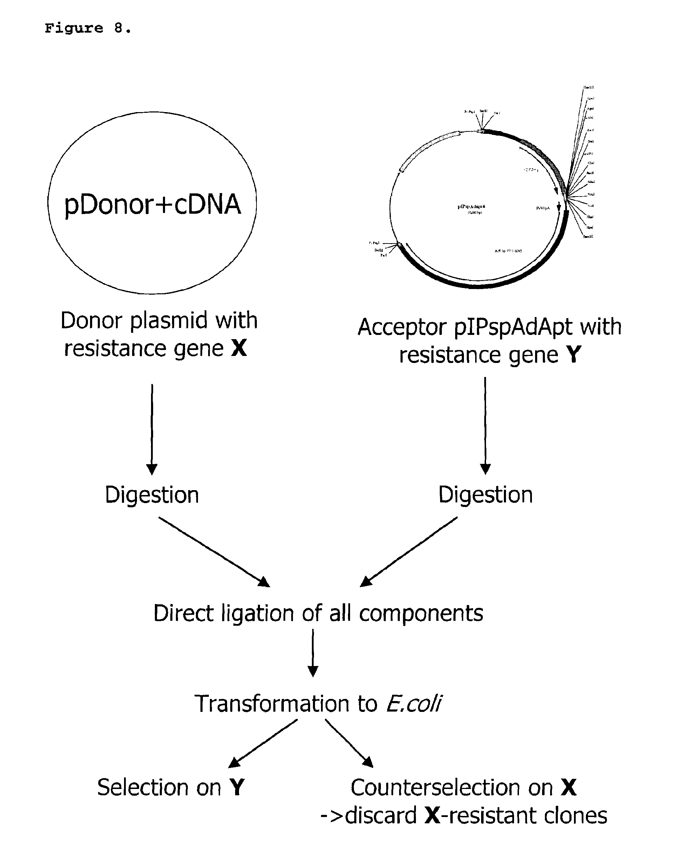

15 minicultures were inoculated from colonies that did not grow on ampicillin plates. Miniprep analysis showed that 100% of the clones derived from batch 1 of the universal pIPspAdapt10/Zeo-lacZpart acceptor vector and 93% of the clones derived from batch 2 of the universal pIPspAdapt10/Zeo-lacZpart acceptor vector contained the correctly swapped EF1α insert. This shows that the swap procedure, in combination with counter selection for the donor vector (Figure 8), is a very efficient method for the conversion of a cDNA insert from a donor vector to an acceptor vector.

### Description of Figures

Figure 1: Schematic representation of pIPspAdApt8.
Figure 2: Schematic representation of pIPspAdApt10.
Figure 3: Schematic representation of adenoviral acceptor vectors pIPspAdApt8/Zeo (A) and pIPspAdApt10/Zeo (B).
Figure 4: Schematic representation of pIPspAdApt6-lacZ.
Figure 5: Schematic representation of universal adenoviral acceptor vector pIPspAdApt10/Zeo-lacZpart.
Figure 6: Schematic representation of pIPspAdApt6-EGFP.
Figure 7: Schematic representation of pCLIP-Pac1-EF1α.
Figure 8: Schematic representation of the swapping/counter selection cloning system. Both the donor vector and the (adenoviral) acceptor vector are digested with compatible restriction endonucleases, (inactivated) reaction mixtures are directly used for the ligation step from which an aliquot is transformed into *E. coli.* Transformants are selected on antibiotics for which the acceptor vector confers resistance (Y). Transformants that grow on the antibiotics for which the donor vector confers resistance (X) are counter-selected and discarded. Transformants that grow on Y and not on X are used for vector nucleic acid isolations.

## Claims

1. Method for cloning a sample nucleic acid from a donor vector conferring resistance to one or more antibiotics of a first type to an acceptor vector conferring resistance to one or more antibiotics of a second type without purification of said sample nucleic acid from said donor vector, comprising the following steps:
(a) digesting said donor vector comprising said sample nucleic acid to be cloned with at least one restriction endonuclease which releases said sample nucleic acid from said donor vector,
(b) digesting said acceptor vector with at least one compatible restriction endonuclease for cloning said sample nucleic acid,
(c) adding together the product of step (a) and (b) in a ligation reaction mixture,
(d) transformation of said mixture of step (c) into a suitable host,
(e) plating said host cells of step (d) on plates containing selective growth medium containing one or more antibiotics of the second type, and,
(f) possibly isolating vector nucleic acid containing said sample nucleic acid from the host cells resulting after step (e).

2. Method according to claim 1 wherein step (e) is followed by a further step comprising plating said selected host cells of step (e) on plates containing selective growth medium containing the antibiotic for which the donor vector confers resistance and discarding colonies which still grow under these counter selection conditions.

3. Method according to any of claims 1 or 2 wherein said sample nucleic acid is part of a set or library of sample nucleic acids that are individually cloned into the donor vector.

4. Method according to any of claim 1 to 3 which is part of an automated or high-throughput procedure.

5. Method according to any of claims 1 to 4, wherein said donor vector contains an ampicillin-resistance gene.

6. Method according to any of claims 1 to 4, wherein said acceptor vector contains a zeocin-resistance gene.

7. Method according to any of claims 1 to 6 wherein said acceptor vector is an expression vector.

8. Method according to any of claims 1 to 7 wherein said acceptor vector is a viral expression vector.

9. Method according to any of claims 1 to 8 wherein said acceptor vector is a retroviral vector.

10. Method according to any of claims 1 to 8 wherein said acceptor vector is an adenoviral vector.

11. Method according to claim 10 wherein said adenoviral vector is an adenoviral adapter vector which contains the left ITR and part of the E2B region, and in which the E1 region has been exchanged for a mammalian promoter, a polylinker sequence, and a polyadenylation signal.

12. Method according to claim 11 wherein said adenoviral vector is pIPspAdApt10/Zeo-lacZpart as shown in Figure 5.

13. Adenoviral vector as defined in claim 12.
